# EUROPEAN PATENT APPLICATION

(11) **EP 4 821 453 A1**
(43) Date of publication of application: **14.10.2026**
(21) Application number: 25168884.2
(22) Date of filing: 07.04.2025
(51) Int. Cl.: A61B 18/20

(54) **COMPUTER-IMPLEMENTED METHOD, SKIN TREATMENT DEVICE, COMPUTER PROGRAM PRODUCT, AND PROCESSING DEVICE**

(71) Applicant: Koninklijke Philips N.V., 5656 AE Eindhoven (NL)
(72) Inventor: DE VRIES, Siebrand Leen, Eindhoven (NL)
(74) Representative: Philips Intellectual Property & Standards

(57) **Abstract**

There is provided a computer-implemented method of providing user guidance for a skin treatment device to perform skin treatment. The computer-implemented method comprises: establishing a connection between the skin treatment device and a processing device performing the computer-implemented method; determining a start of a treatment with the skin treatment device at a first point of time; determining the connection is interrupted after the first point of time; re-establishing the connection; determining whether a time period between the first point of time and a second point of time exceeds a threshold, wherein the second point of time is when the connection has been re-established; in case the time period does not exceed the threshold, receiving use data from the skin treatment device, and adapting the user guidance based on the use data; in case the time period exceeds the threshold, not adapting the user guidance based on the use data, wherein the use data is representative of one or more skin treatment sessions performed with the skin treatment device during the time period; generating a guidance signal representative of the user guidance.

## Description

### FIELD OF THE INVENTION

The invention relates to a computer-implemented method of providing user guidance for a skin treatment device to perform skin treatment. Further, the invention relates to a skin treatment device adapted to perform skin treatment, especially an intense pulsed light (IPL) device. Further, the invention relates to a computer program product and a processing device to carry out the computer-implemented method.

### BACKGROUND OF THE INVENTION

Photo-epilation is well known for hair removal and hair growth reduction. Home-use consumer devices are commercially available, such as the Lumea (TM) of Philips (TM). Home-use devices typically use Intense Pulsed Light technology (IPL) from e.g. a Xenon flash lamp at a relatively low fluence (up to 6.5 J/cm²), as compared to professional devices for permanent photo-epilation, that use fluences in excess of 10 J/cm².

IPL technology uses a high-powered, hand-held, flash lamp to deliver an intense, visible, broad-spectrum pulse of light, generally in the visible spectral range of 400 to 1200 nm. Cutoff filters are for example used to selectively filter out shorter wavelengths, especially potentially damaging ultra violet light. The resulting light has a spectral range that targets specific structures and chromophores, in particular the melanin pigment in hair. The light absorbed by melanin in the hair and hair matrix generates heat that puts the hair follicles in a sleep state. When the user uses the IPL device according to a treatment schedule in which treatments are repeated in intervals of 2 to 4 weeks, a long-lasting hair reduction result is obtained.

To guide the user through the treatment schedule, an app on a mobile phone is used to create user guidance for the user. The app is able to receive information about the use of the IPL device. The user guidance is adapted based on if and when the user uses the IPL device. The app guides the user to the next treatment based on the treatments schedule and the received information about the use of the IPL device.

### SUMMARY OF THE INVENTION

However, sometimes a user uses the IPL device without using the app. For example, the user is out of range of the mobile device executing the app, or the mobile device is unable to execute the app, such as when executing a telephone call or displaying a video. As the app is not aware of some use of the IPL device, the app provides insufficient guidance. Due to the insufficient guidance, the user uses the IPL device for more treatments or for less treatments than is required to obtain a desired depilation.

It is an objective of the invention to improve the user guidance for a skin treatment device to perform skin treatment.

According to a first aspect, there is provided a computer-implemented method of providing user guidance for a skin treatment device to perform skin treatment. The computer-implemented method comprises establishing a connection between the skin treatment device and a processing device performing the computer-implemented method. The computer-implemented method comprises determining a start of a treatment with the skin treatment device at a first point of time. The computer-implemented method comprises determining the connection is interrupted after the first point of time. The computer-implemented method comprises re-establishing the connection. The computer-implemented method comprises determining whether a time period between the first point of time and a second point of time exceeds a threshold. The second point of time is when the connection has been re-established. The computer-implemented method comprises, in case the time period does not exceed the threshold, receiving use data from the skin treatment device, and adapting the user guidance based on the use data. The computer-implemented method comprises in case the time period exceeds the threshold, not adapting the user guidance based on the use data. The use data is representative of one or more skin treatment sessions performed with the skin treatment device while the connection was interrupted. The computer-implemented method comprises generating a guidance signal representative of the user guidance.

The time period is between when the skin treatment device started the treatment and when the connection between the skin treatment device and the processing device is re-established after the connection was interrupted. If the time period is within the threshold, the use data received from the skin treatment device relates to use of the skin treatment device within a certain time period. As this certain time period is relatively short, the use of the skin treatment device while the connection is interrupted is seen by the user guidance as part of the treatment that was already started. So the user is able to perform part of the treatment without using the processing device, while achieving proper skin treatment.

If the time period is longer than the threshold, there may be too much uncertainty at which time the use data was created. The use data may have been created shortly after the connection was interrupted, so a long time before the connection was re-established, or the use data may have been created shortly before the connection was re-established. Because of this uncertainty, continuing the treatment after the connection is re-established may not lead to acceptable skin treatment results. Therefore, if the time period is longer than the threshold, the user guidance is not adapted, so any use of the skin treatment device while the connection is interrupted is ignored by the user guidance. The user is guided by the un-adapted user guidance to ensure a desired skin treatment result.

This results in an improved user guidance for the skin treatment device to perform skin treatment.

Providing the computer-implemented method according to the first aspect is especially beneficial for a skin treatment device that does not have a real time clock to determine time in case the skin treatment device is switched off. For example, some skin treatment devices, such as IPL devices, have a power cord to receive sufficient electrical energy to perform skin treatment. Those skin treatment devices do not need a battery to perform the skin treatment as the electrical energy is provided via the power cord. However, because those skin treatment devices do not have a battery, they cannot operate a real time clock when unplugged or switched off. Adding a battery to operate a real time clock would add costs to the device. According to the computer-implemented method according to the first aspect, the processing device is able to determine whether more or less time than the threshold has passed since the start of the treatment, and whether to adapt the user guidance based on the use data without the skin treatment device providing information about the exact time the use data was generated. As a result, the skin treatment device does not require a real time clock nor a battery for the real time clock, which reduces the costs, size and complexity of the skin treatment device.

A skin treatment device is, for example, an intense pulsed light (IPL) device adapted to provide IPL treatment. For example, the IPL treatment comprises photo epilation. For example, the skin treatment device is an epilation device, such as an IPL device or a mechanical epilator. For example, the skin treatment device is a hair grooming device, such as a shaver or a hair clipper.

The connection between the skin treatment device and a processing device performing the computer-implemented method is, for example, a wireless connection. The wireless connection is, for example, via Bluetooth or Wi-Fi or any other type of wireless communication. For example, when the connection is established, the processing device receives a signal from the skin treatment device. The signal is, for example, in response to a signal sent by the processing device. The signal is, for example, the signal comprising the use data. The signal is, for example, a signal generated by the skin treatment device in response to being switched on.

The processing device is a device that is separate from the skin treatment device. For example, the processing device has a power source separate from the skin treatment device. For example, the processing device has a processor to perform the computer-implemented method. For example, the processing device is adapted to communicate, such as via internet, to one or more external processors. The external processors are separated from the processing device. The processing device is adapted to perform the computer-implemented method in cooperation with the one or more external processors.

The first point of time is representative of the time at which the treatment with the skin treatment device is started. For example, the treatment is the treatment of a body part. For example, the treatment is started when the skin treatment device is brought into contact with the skin for the first time of the treatment. For example, in case the skin treatment device is an IPL device, the treatment is started when the IPL device provides the first flash of the treatment to the body part. For example, the first point of time is when the user operates the processing device to indicate to the processing device the user intends to start a new treatment.

The time period is between the first point of time and the second point of time.

The second point of time is, for example, the time the connection between the skin treatment device and the processing device is re-established. The second point of time value is, for example, when the user switches on the skin treatment device and starts the computer-implemented method with the processing device. The second point of time value is, for example, when the user plugs the skin treatment device into a power outlet, and brings the skin treatment device and the processing device into an operational range from each other.

The threshold represents a maximum amount of time between the first point of time and the second point of time that allows for the use data that was generated while the connection was interrupted, to be used to adapt the user guidance. The threshold is, for example, 12 hours, or 24 hours, or 36 hours, or 48 hours, or 3 days.

A treatment comprises one or more skin treatment sessions. For example, a treatment comprises a first skin treatment session after the connection is established, a second skin treatment session that is after the first skin treatment session when the connection is interrupted, and a third skin treatment session when the connection is re-established. The second skin treatment session is part of the same treatment as the first skin treatment session in case the time period does not exceed the threshold. In case the time period exceeds the threshold, the second skin treatment session would be a session in a new treatment. For example, a new skin treatment session is started after the user switches off and on the skin treatment device.

The use data is representative of one or more skin treatment sessions performed with the skin treatment device while the connection is interrupted. For example, the use data comprises information relating to how long the skin treatment device was used, or how many skin treatment sessions in the treatment were performed by the skin treatment device, or which attachment was used. For example, for an IPL device, the use data comprises the number of flashes the IPL device provided, or the intensity level at which the flashes were provided, or which attachment was used.

The user guidance is adapted, for example, by marking a planned treatment as completed. The user guidance then guides the user to the next planned treatment. The user guidance is adapted, for example, for guiding the user to another body part than the body part that was treated according to the use data. The user guidance is adapted, for example, to indicate the total time the treatment took. The total time provides the user with guidance on whether the skin was overtreated or undertreated. For an IPL device, the user guidance is adapted, for example, to indicate the total number of flashes in the treatment. The total number of flashes provides the user with guidance on whether the skin was overtreated or undertreated.

The guidance signal is representative of the user guidance. For example, the guidance signal is for causing a display to indicate when the user should perform the next treatment according to the treatment schedule. For example, the guidance signal is for causing a display to indicate a body part that is to be treated next. For example, the guidance signal is for causing a display to indicate the total time of the treatment, optionally in comparison with a default total time of treatment. For example, the guidance signal is for causing a display to indicate the total number of flashes, optionally in comparison with a default total number of flashes.

In an embodiment, each of the skin treatment sessions starts when the skin treatment device is set to start to perform skin treatment based on at least one device setting. Each of the skin treatment sessions ends when at least one device setting changes.

According to this embodiment, a skin treatment session is defined as a part of a treatment during which the device settings of the skin treatment device remain the same. Different device settings may have different effects on the skin. By making use of skin treatment sessions defined in this way, the user guidance is able to provide improved guidance.

In an embodiment, the at least one device setting comprises an on/off setting, an intensity level to perform the skin treatment, or an attachment attached to the skin treatment device.

According to this embodiment, a session is defined in one or more of the following ways. In case the device setting comprises an on/off setting, a new skin treatment session is started when the device is switched on. The skin treatment session ends when the device is switched off. In case the skin treatment device has no real time clock, there may be no information how much time there is between sessions that took place while the connection was interrupted. However, the number of skin treatment sessions provides information for the user guidance, as the number of sessions indicates how many times the user switched the skin treatment device on and off. The user guidance is updated accordingly. In case the device setting comprises an intensity level to perform the skin treatment, the use data indicates the intensity level for each session. This provides information for the user guidance about with how much intensity the skin is treated. The user guidance is updated accordingly. In case the device setting comprises the attachment attached to the skin treatment device, the use data indicates which session is performed with which attachment. For example, different attachments are applied to different body parts. This provides information to the user guidance which body parts have been treated. For example, different attachments may affect the skin differently. This provides information to the user guidance about how the skin was treated. For example, the use data comprises information about multiple device settings, e.g., both the intensity level as well as the attachment used.

In an embodiment, the skin treatment device is an intense pulsed light (IPL) device adapted to perform IPL treatment by providing IPL flashes to the skin. The use data comprises one or more of: a number of provided IPL flashes, a duration of the IPL treatment, or an intensity of the IPL flashes.

According to the embodiment, the IPL device provides IPL flashes to the skin. For example, the IPL device comprises a light source, such as a flash lamp, to provide the IPL flashes. The IPL flashes provide treatment for the skin. For example, the IPL flashes cause depilation of the skin. For example, the IPL flashes cause other effects on the skin, such as skin rejuvenation. The number of provided IPL flashes, the duration of the IPL treatment, and the intensity of the IPL flashes provide information about the amount of treatment performed on the skin. Based on that information, the user guidance is adapted.

In an embodiment, the use data comprises an indication of an attachment used to perform the skin treatment. The attachment is one of multiple attachments adapted to connect to the skin treatment device. The multiple attachments are adapted to perform the skin treatment to different body parts.

According to the embodiment, the skin treatment device can be provided with multiple attachments to provide skin treatment to the skin. The attachments are adapted to be used on different body parts. For example, one attachment is for providing skin treatment to the face, whereas another attachment is for providing skin treatment to the legs. To properly provide skin treatment to the different body parts, the attachments provide skin treatment differently. For example, an attachment for treating the face has a small operating area to be used on a small area of skin. This is helpful to move the attachment around the eyes and mouth and ears. For example, the attachment for the face provides skin treatment with a low intensity. For example, an attachment for treating the legs has a large operating area to be used on a large area of skin. This is helpful to cover the whole of the legs in a shorter amount of time. For example, the attachment for the legs provides skin treatment with a high intensity. By providing information with the use data about which attachment is used, the user guidance can be updated on how the skin is treated.

In an embodiment, establishing the connection comprises establishing the connection via a wireless connection between the skin treatment device and the processing device.

According to this embodiment, the skin treatment device and the processing device are in connection with each other via a wireless connection, such as Bluetooth, Wi-Fi, or any other suitable wireless communication means.

In an embodiment, the processing device is a mobile device.

In an embodiment, the threshold is less than 48 hours, for example 24 hours.

According to this embodiment, the user is able to continue the treatment after the connection is re-established in case the treatment started less than 48 hours before. For applications such as photo-epilation, effective depilation is achieved in case the treatment is continued within 48 hours. In case there is more than 48 hours after the start of the treatment, it is uncertain whether effective depilation can be achieved by continuing the treatment. In that case, a new treatment is started to ensure effective depilation. By using a threshold of 24 hours, this is even more certainty that effective depilation may be obtained.

In an embodiment, the user guidance comprises guidance for the user about one or more of when to use the skin treatment device to perform skin treatment, on which body part to use the skin treatment device to perform skin treatment, which intensity setting to use, and which attachment to use.

For some skin treatments, it is important that treatment is performed at the correct intervals. For example, in photo-epilation, the skin treatment is most effective in case hair is treated in the correct hair growth phase. In case the time period does not exceed the threshold, most of the hairs are in the same hair growth phase as at the start of the treatment, so the treatment can be continued. In case the time period exceeds the threshold, most of the hairs may no longer be in the same hair growth phase as at the start of the treatment, so a new treatment is started to ensure effective hair removal. By guiding the user when to use the skin treatment device, improved skin treatment is obtained. By providing guidance on which body part to use the skin treatment device, the user is guided to, for example, continue treatment of the body part or start treatment of another body part. This way, effective treatment of all body parts is obtained. By providing guidance about which intensity setting to use, the user guidance guides the user to, for example, use the same intensity for the skin treatment device as at the start of the treatment, or to use a different intensity to start a new treatment. By providing guidance about which attachment to use, the user guidance guides the user to, for example, use the same attachment for the skin treatment device as at the start of the treatment, or to use a different attachment to start a new treatment.

In an embodiment, the computer-implemented method comprises sending a reset signal to the skin treatment device. The reset signal causes the skin treatment device to remove the use data from the skin treatment device.

According to the embodiment, the reset signal is sent to the skin treatment device to remove the use data from the skin treatment device. By removing the use data, storage space for new use data becomes available. This way, only limited storage space, e.g., a memory, for use data is sufficient to store the use data generated during the time period in case the connection is interrupted. For example, the method comprises sending the reset signal in response to determining that the time period between the first point of time and the second point of time exceeds the threshold. For example, the second point of time is at the moment the connection is re-established.

In an embodiment, the computer-implemented method comprises receiving user input representative of whether the user wants to start a new treatment. In case the user input is representative of the user wanting to start the new skin treatment, the computer-implemented method comprises not adapting the user guidance based on the use data.

According to the embodiment, the user is able to determine whether a new treatment is started, even though the previous treatment could be continued. The user indicates via the user input that the start of a new treatment is desired. In response, the user guidance is not adapted based on the use data. Instead, the original user guidance is provided, which disregards the use of the skin treatment device while the connection was interrupted. For example, the user wants to start a new treatment to ensure the best treatment outcome, even though this may take more time and effort than continuing the previous treatment.

In a second aspect of the invention, there is provided a skin treatment device adapted to perform skin treatment. The skin treatment device comprises a skin treatment unit adapted to perform the skin treatment. The skin treatment device comprises a use data collection unit adapted to collect use data representative of one or more skin treatment sessions performed with the skin treatment device. The skin treatment device comprises a use data storage unit adapted to store the use data collected by the use collection unit. The skin treatment device comprises a connection unit adapted to connect to a processing device adapted to perform the computer-implemented method according to the first aspect of the invention.

In an embodiment, the skin treatment device is an intense pulsed light (IPL) device adapted to perform IPL treatment by providing IPL flashes to the skin. The skin treatment unit comprises an IPL light source adapted to generate the IPL flashes. The use data comprises one or more of: a number of provided IPL flashes, a duration of the IPL treatment, an intensity of the IPL flashes, and an indication of an attachment used to perform the IPL treatment. The attachment is one of multiple attachments adapted to connect to the IPL device. The multiple attachments are adapted to propagate the IPL flashes to different body parts.

For example, the skin treatment unit comprises a treatment light source adapted to provide the IPL flashes. For example, the treatment light source is adapted to generate the IPL flashes at a high intensity for a short duration, such as for less than 100 ms or less than 50 ms or less than 10 ms or less than 8 ms. The intensity of the IPL flashes is high enough to perform a treatment on the skin. Such treatment is, for example, hair removal or photo-rejuvenation or vein treatment or acne treatment. For example, the treatment light source comprises one or more optical filters. For example, the treatment light source comprises an optical filter to prevent light with a potentially damaging wavelength, such as UV light, from being transmitted to the skin. For example, the treatment light source comprises a Xenon flash lamp, or a laser, or multiple lasers, a LED, or multiple LEDs. For example, the treatment light source is adapted to provide IPL flashes with wavelengths in the range of 530-1200 nanometers.

In a third aspect of the invention, there is provided a computer program product comprising instructions, which when executed by a processor, cause the computer-implemented method of the first aspect to be carried out.

In a fourth aspect of the invention, there is provided a processing device, comprising a processor adapted to execute the computer program product of the third aspect.

### BRIEF DESCRIPTION OF THE DRAWINGS

Exemplary embodiments will now be described, by way of example only, with reference to the following Figures, in which:
FIG. 1 depicts a skin treatment device according to a first embodiment;
FIG. 2 depicts a cross-section of the skin treatment device according to the first embodiment;
FIG. 3 depicts a computer-implemented method according to the first embodiment;
FIGs 4-7 depict the operation of the computer-implemented method according to the first embodiment in different situations;
FIG. 8 depicts the user guidance according to the first embodiment;
FIG. 9 depicts part of the processing device according to a second embodiment;
FIG. 10 depicts the computer-implemented method according to a third embodiment.

### DETAILED DESCRIPTION OF EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

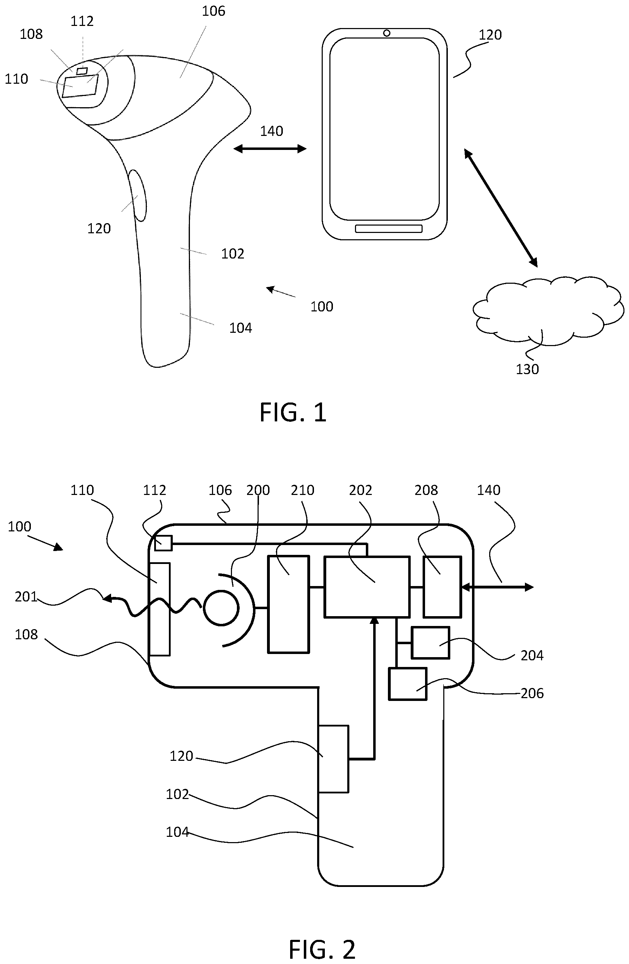

FIGs. 1 and 2 depict a skin treatment device 100 according to a first embodiment. The skin treatment device 100 is adapted to perform skin treatment. In the first embodiment, the skin treatment device 100 is an IPL apparatus. The skin treatment device 100 is adapted to perform skin treatment on a subject, i.e., a person or an animal. The skin treatment device 100 comprises a housing 102 that has a handle portion 104 and a head portion 106. The handle portion 104 is shaped to enable the user to hold the skin treatment device 100. The head portion 106 has a head end 108 that is to be placed into contact with the subject in order for the skin treatment device 100 to perform a treatment on the skin of the subject.

The skin treatment device 100 comprises a skin treatment unit 200. The skin treatment unit 200 is adapted to perform skin treatment. In the first embodiment, the skin treatment device 100 is an intense pulsed light (IPL) device adapted to perform IPL treatment by providing IPL flashes 201 to the skin. The skin treatment unit 200 comprises an IPL light source adapted to generate the IPL flashes 201.

The skin treatment unit 200 is arranged inside the head portion 106. The skin treatment device 100 performs the treatment by providing IPL flashes 201 to the skin via the treatment window. The treatment window 110 is arranged in or on the housing 102 so that the treatment window 110 can be placed adjacent to or on (i.e. in contact with) the skin of the subject. The intensity of the IPL flashes 201 from the skin treatment unit 200 are high enough to perform treatment on the skin or body part adjacent to the treatment window 110.

The skin treatment unit 200 comprises a user control 120 adapted to be operated by the user to activate the skin treatment unit 200. When the user activates the skin treatment unit 200 via the user control 120, the skin treatment unit 200 is able to generate an IPL flash 201. However, the skin treatment device 100 is, for example, configured not to generate an IPL flash 201 in some situations, even if the user operates the user control 120. Some of these situations are explained below. The user control 120 may be in the form of a switch, a button, or a touch pad.

The skin treatment device 100 comprises a sensor 112. For example, the sensor 112 is adapted to measure a characteristic of the skin, such as skin tone. For example, the sensor 112 is adapted to measure whether skin is present near the head end 108. In case skin is present near the head end 108, the skin treatment device 100 is adapted to generate an IPL flash 201. In case skin is not present near the head end 108, the skin treatment device 100 is adapted not to generate an IPL flash 201, even when the user operates the user control 120.

The skin treatment device 100 comprises a device processor 202, a use data collection unit 204, a use data storage unit 206, a connection unit 208, and a driver 210. The device processor 202 is configured to operate the skin treatment device 100. The device processor 202 is configured to receive a user signal from the user control 120 representative of the user operating the user control 120. In response to the user signal, the device processor 202 controls the driver 210. The driver 210 provides electrical power to the skin treatment unit 200 to generate an IPL flash 201. The device processor 202 is configured to receive a sensor signal from the sensor 112. For example, the device processor 202 is configured to adapt the control of the driver 210 based on the sensor signal. For example, the device processor 202 is configured to adapt an intensity of the IPL flash 201 based on the sensor signal.

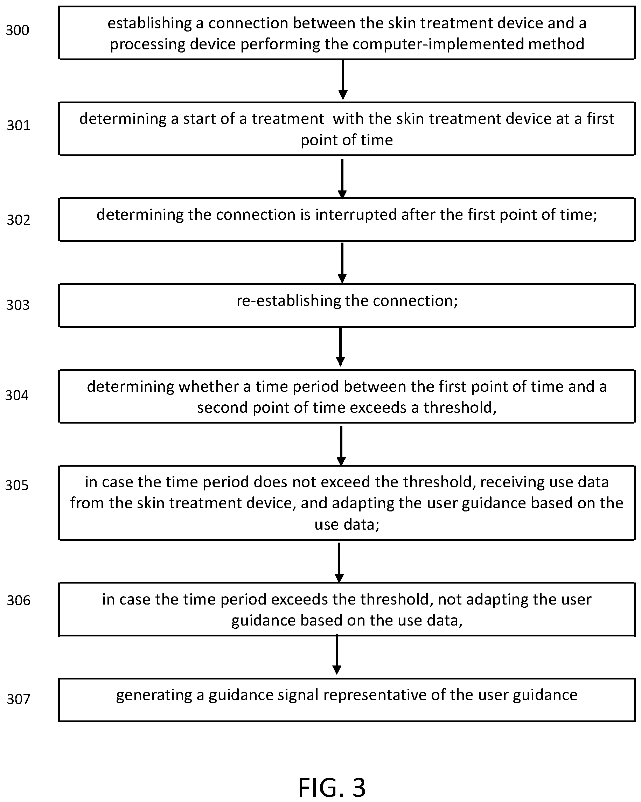

The use data collection unit 204 is adapted to collect use data representative of one or more skin treatment sessions performed with the skin treatment device 100. The use data storage unit 206 is adapted to store the use data collected by the use collection unit. For example, the use data collection unit 204 and/or the use data storage unit 206 are integrated in the device processor 202. The connection unit 208 is adapted to connect to a processing device adapted to perform a computer-implemented method. For example, the processing device is mobile device 120. The mobile device 120 is configured to perform the computer-implemented method, as will be explained further with reference to FIG. 3.

FIG. 3 depicts the computer-implemented method according to the first embodiment. The computer-implemented method is for providing user guidance for the skin treatment device 100 to perform skin treatment. The computer-implemented method comprises at 300, establishing a connection 140 between the skin treatment device 100 and a processing device performing the computer-implemented method. In the first embodiment the processing device performing the computer-implemented method is the mobile device 120. The computer-implemented method comprises at 301, determining a start of a treatment with the skin treatment device 100 at a first point of time t1. The computer-implemented method comprises at 302, determining the connection 140 is interrupted after the first point of time t1. The computer-implemented method comprises at 303, re-establishing the connection 140. The computer-implemented method comprises at 304, determining whether a time period between the first point of time t1 and a second point of time t2 exceeds a threshold. The second point of time is when the connection 140 has been re-established. The computer-implemented method comprises at 305, in case the time period does not exceed the threshold, receiving use data from the skin treatment device 100, and adapting the user guidance based on the use data. The computer-implemented method comprises at 306, in case the time period exceeds the threshold, not adapting the user guidance based on the use data. The use data is representative of one or more skin treatment sessions performed with the skin treatment device 100 during the time period. The computer-implemented method comprises at 307, generating a guidance signal representative of the user guidance.

For example, the guidance signal cause the display of the mobile device 120 to show the user guidance. For example, the user guidance guides the user to move the skin treatment device 100 to a certain body part, to use the skin treatment device 100 at a certain setting, to use the skin treatment device 100 at a certain time.

The computer-implemented method establishes the connection 140 via a wireless connection between the skin treatment device 100 and the processing device, i.e., the mobile device 120.

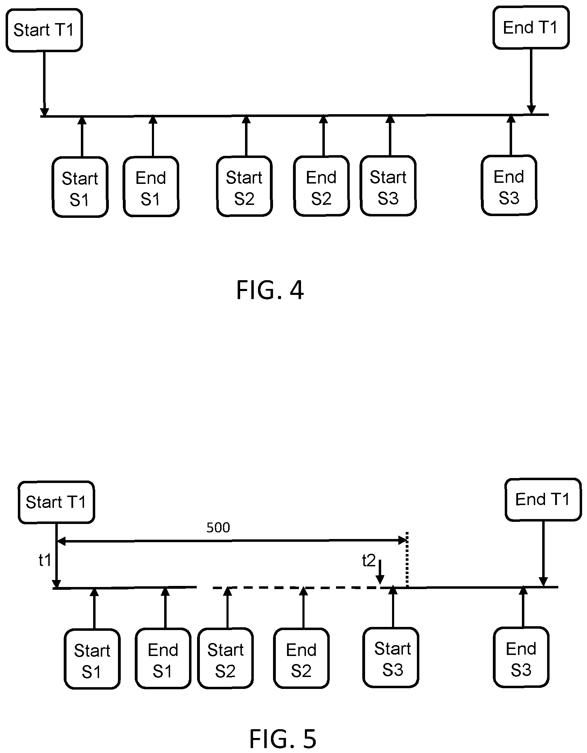

FIGs 4-6 depict the operation of the computer-implemented method according to the first embodiment in different situations.

FIG. 4 depicts the operation of the computer-implemented method in a first situation. In the first situation, the connection 140 between the skin treatment device 100 and the mobile device 120 is established and is not interrupted. The user indicates to the mobile device 120 that the user wants to start a first treatment T1. Then the user operates the skin treatment device 100 to start a first skin treatment session S1. The first skin treatment session S1 is part of the first treatment T1. During the first skin treatment session S1, the user operates the skin treatment device 100 to perform skin treatment on the skin, for example by operating the user control 120. Later, the user ends the first skin treatment session S1.

Later, the user operates the skin treatment device 100 to start a second skin treatment session S2. The second skin treatment session S2 is part of the first treatment T1. Since the connection 140 between the skin treatment device 100 and the mobile device 120 was established during the first skin treatment session S1, and is established at the start of the second skin treatment session S2, the mobile device 120 is able to determine the time from the start of the first treatment T1, by using a real time clock. This allows the user to continue the first treatment T1 by starting the second skin treatment session S2. During the second skin treatment session S2, the user operates the skin treatment device 100 to perform skin treatment on the skin, for example by operating the user control 120. Later, the user ends the second skin treatment session S2.

Later, the user operates the skin treatment device 100 to start a third skin treatment session S3. The third session S3 is part of the first treatment T1. Since the connection 140 between the skin treatment device 100 and the mobile device 120 was established during the first skin treatment session S1 and the second skin treatment session S2, and is established at the start of the third skin treatment session S3, the mobile device 120 is able to determine the time from the start of the first treatment T1, by using a real time clock. This allows the user to continue the first treatment T1 by starting the third skin treatment session S3. During the third skin treatment session S3, the user operates the skin treatment device 100 to perform skin treatment on the skin, for example by operating the user control 120. Later, the user ends the third skin treatment session S3. At the end of the third skin treatment session S3, the first treatment T1 is completed and ended by the computer-implemented method. In case the user starts a new skin treatment session afterwards, the new skin treatment session is in a new treatment.

In the situation above, the connection 140 was established while the user operates the skin treatment device 100. As a result, all use data from the skin treatment device 100 can be transferred directly to the mobile device 120. The processor of the mobile device 120 is able to adapt the user guidance based on the use data.

FIG. 5 depicts the operation of the computer-implemented method in a second situation. In the second situation, the user indicates to the mobile device that the user wants to start the first treatment T1. Then the user operates the skin treatment device 100 to start the first skin treatment session S1. The first skin treatment session S1 is part of the first treatment T1. During the first skin treatment session S1, the user operates the skin treatment device 100 to perform skin treatment on the skin, for example by operating the user control 120. Later, the user ends the first skin treatment session S1. The connection 140 between the skin treatment device 100 and the mobile device 120 is established before the first skin treatment session S1 and is not interrupted during the first skin treatment session S1, similar to the first situation.

After the first skin treatment session S1 ended, the connection 140 is interrupted, as indicated with the dashed line. For example, the mobile device 120 is switched off, or is out of range of the skin treatment device 100. When the user performs the second skin treatment session S2, there is no connection 140 between the skin treatment device 100 and the mobile device 120.

The skin treatment device 100 collects the use data during the second skin treatment session S2 and stores the use data in the use data storage unit 206.

Later, the connection 140 between the skin treatment device 100 and the mobile device 120 is re-established. The moment that the connection 140 is re-established is at the second point of time t2. By using the real time clock, the mobile device performs the computer-implemented method and determines whether the time period between the first point of time t1 and the second point of time t2 exceeds a threshold 500.

In the second situation, the time period does not exceed the threshold 500. Therefore, the use data of the second skin treatment session S2 is used to adapt the user guidance. The user guidance takes the second skin treatment session S2 into account, for example, when planning the third skin treatment session S3 or to determine a device setting for the third skin treatment session S3.

Then, the user starts the third skin treatment session S3. The start of the third skin treatment session S3 is within the time period as defined by the threshold 500 and is therefore part of the first treatment T1. When the third skin treatment session S3 ends, the first treatment T1 is ended. So the first treatment T1 has the first skin treatment session S1, the second skin treatment session S2, and the third skin treatment session S3. In case the user starts a new skin treatment session, the new skin treatment session is part of a new treatment.

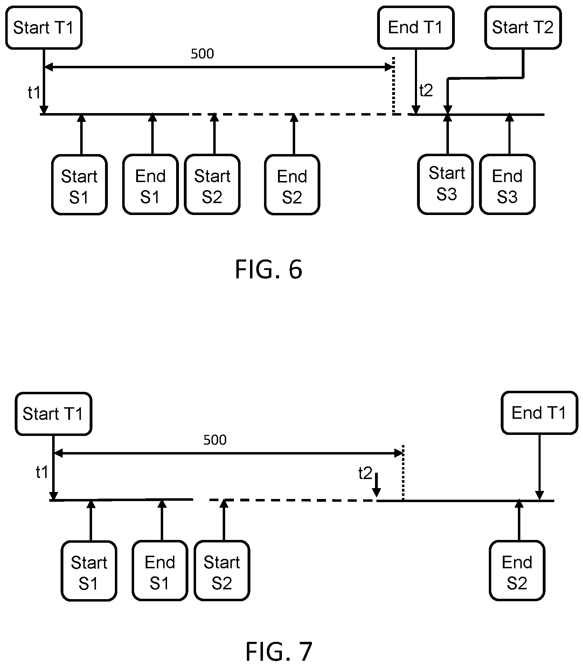

FIG. 6 depicts the operation of the computer-implemented method in a third situation. In the third situation, the user indicates to the mobile device 120 that the user wants to start the first treatment T1. Then the user operates the skin treatment device 100 to start the first skin treatment session S1. The first session S1 is part of the first treatment T1. During the first skin treatment session S1, the user operates the skin treatment device 100 to perform skin treatment on the skin, for example by operating the user control 120. Later, the user ends the first skin treatment session S1. The connection 140 between the skin treatment device 100 and the mobile device 120 is established before the first skin treatment session S1 and is not interrupted during the first session S1, similar to the first situation.

After the first skin treatment session S1 ended, the connection 140 is interrupted, as is indicated by the dashed line. For example, the mobile device 120 is switched off, or is out of range of the skin treatment device 100. When the user starts and performs the second skin treatment session S2, there is no connection 140 between the skin treatment device 100 and the mobile device 120.

The skin treatment device 100 collects the use data during the second session S2 and stores the use data in the use data storage unit 206.

Later, the connection 140 between the skin treatment device 100 and the mobile device 120 is re-established. The moment that the connection 140 is re-established is at the second point of time t2. By using the real time clock, the mobile device 120 performs the computer-implemented method and determines whether the time period between the first point of time t1 and the second point of time t2 exceeds the threshold 500.

In the third situation, the time period exceeds the threshold 500. The second point of time t2 is later than the end of the time period defined by the threshold 500. Therefore, the use data of the second skin treatment session S2 is not used to adapt the user guidance. The user guidance does not take the second skin treatment session S2 into account, for example, when planning the third skin treatment session S3 or to determine a device setting for the third skin treatment session S3. The first treatment T1 ends at the second point of time t2.

The computer-implemented method causes the mobile device 120 to send a reset signal to the skin treatment device 100. The reset signal causes the skin treatment device 100 to remove the use data from the skin treatment device 100.

In the first embodiment, the threshold 500 is less than 48 hours, for example 24 hours.

Then, the user starts the third skin treatment session S3. The third skin treatment session S3 is part of a new treatment, i.e., the second treatment T2. So in the third situation, the first treatment T1 has only the first skin treatment session S1, whereas the second treatment T2 has the third skin treatment session S3.

For example, each of the skin treatment sessions S1-S3 starts when the skin treatment device 100 is set to start to perform skin treatment based on at least one device setting, and ends when at least one device setting changes. For example, the at least one device setting comprises an on/off setting, an intensity level to perform the skin treatment, or an attachment attached to the skin treatment device 100.

So for example, the first skin treatment session S1 starts when the user switches the skin treatment device 100 on. The first skin treatment session S1 ends when the user changes an intensity setting of the IPL flashes 201. The second skin treatment session S2 begins when the user operates the user control to generate IPL flashes 201 with the skin treatment device 100 with the changed intensity setting. The second skin treatment session S2 ends when the user changes an attachment of the skin treatment device 100. The third skin treatment session S3 starts when the user operates the user control to generate IPL flashes 201 with the skin treatment device 100 with the new attachment.

The attachments are, for example, to provide the IPL flashes 201 to the skin. For example, the attachments are adapted to connect to the skin treatment device 100. The attachments are adapted to perform the skin treatment to different body parts. For example, the use data comprises an indication of which attachment is used to perform the skin treatment.

In another embodiment, the skin treatment device 100 is an epilator. The skin treatment unit 200 comprises tweezers adapted to grab and pluck hair from the skin. For example, the attachments for the epilator have different contact areas for contacting the skin. For example, the attachments for the epilator operate the tweezers at different speeds. For example, the attachments for the epilator have tweezers with different sizes adapted to grab and pluck different types or sizes of hairs.

In the first embodiment, the processing device is a mobile device 120, such as a mobile phone or a tablet. However, in another embodiment, the processing device is different from a mobile device 120, but is, for example, a personal computer, a laptop, or a server. The processing device has a processor to cause the computer-implemented method to be carried out. The complete computer-implemented method is, for example, carried out by the processor of the processing device. Alternatively, the processing device is configured to connect to other, distant processors, such as in the cloud 130, see FIG. 1. The processing device is configured to have part of the computer-implemented method carried out by the distant processors.

For example, the skin treatment device 100 is an intense pulsed light (IPL) device adapted to perform IPL treatment by providing IPL flashes 201 to the skin. The use data comprises one or more of: a number of provided IPL flashes 201, a duration of the IPL treatment, or an intensity of the IPL flashes 201.

FIG. 7 depicts the operation of the computer-implemented method in a fourth situation. In the fourth situation, the user indicates to the mobile device that the user wants to start the first treatment T1. Then the user operates the skin treatment device 100 to start the first skin treatment session S1. The first skin treatment session S1 is part of the first treatment T1. During the first skin treatment session S1, the user operates the skin treatment device 100 to perform skin treatment on the skin, for example by operating the user control 120. Later, the user ends the first skin treatment session S1. The connection 140 between the skin treatment device 100 and the mobile device 120 is established before the first skin treatment session S1 and is not interrupted during the first skin treatment session S1, similar to the first situation.

After the first skin treatment session S1 ended, the connection 140 is interrupted, as indicated with the dashed line. For example, the mobile device 120 is switched off, or is out of range of the skin treatment device 100. When the user starts the second skin treatment session S2, there is no connection 140 between the skin treatment device 100 and the mobile device 120.

The skin treatment device 100 collects the use data during the second skin treatment session S2 and stores the use data in the use data storage unit 206.

Later, while the user is still using the skin treatment device 100 during the second skin treatment session S2, the connection 140 between the skin treatment device 100 and the mobile device 120 is re-established. The moment that the connection 140 is re-established is at the second point of time t2. By using the real time clock, the mobile device performs the computer-implemented method and determines whether the time period between the first point of time t1 and the second point of time t2 exceeds a threshold 500.

In the fourth situation, the time period does not exceed the threshold 500. Therefore, the use data of the second skin treatment session S2 is used to adapt the user guidance. The user guidance takes the second skin treatment session S2 into account, for example, when planning the third skin treatment session S3 or to determine a device setting for the third skin treatment session S3.

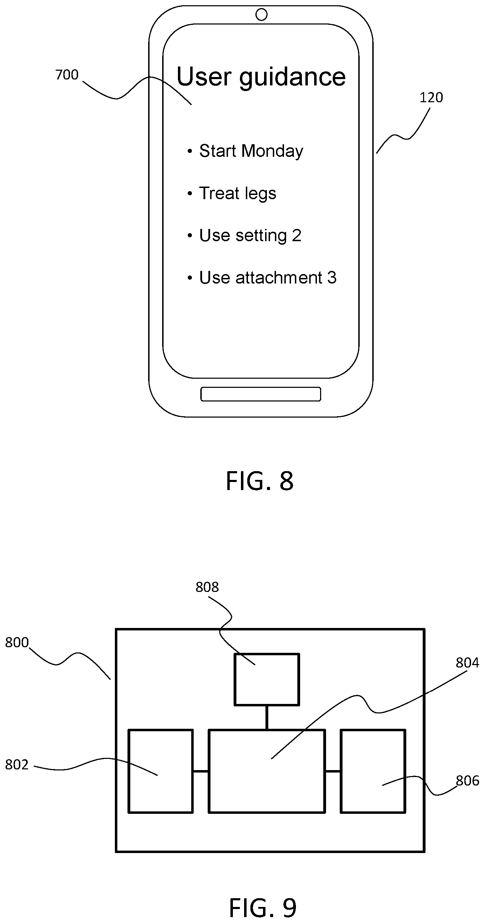

FIG. 8 depicts the user guidance according to the first embodiment. The user guidance comprises guidance 700 for the user about when to use the skin treatment device 100 to perform skin treatment. In this example, the user is guided to start on Monday. The user guidance comprises guidance 700 for the user about on which body part to use the skin treatment device 100 to perform skin treatment. The user is guided to treat the legs. The user guidance comprises guidance 700 for the user about which intensity setting to use. In this example, the user is guided to use intensity setting 2. For example, the intensity setting 2 provides IPL flashes 201 with a higher intensity than intensity setting 1, but with a lower intensity than intensity setting 3. The user guidance comprises guidance 700 for the user about which attachment to use. In this example, the user is guided to use attachment 3. For example, there are three attachments: attachment 1 adapted to provide treatment to the face, attachment 2 adapted to provide treatment to arm pits, and attachment 3 adapted to provide treatment to the legs.

In an embodiment, the computer-implemented method comprises receiving user input representative of whether the user wants to start a new treatment. In case the user input is representative of the user wanting to start the new treatment, the user guidance is not adapted based on the use data.

FIG. 9 depicts part of a processing device 800 according to a second embodiment. The second embodiment has, for example, the same elements as the first embodiment, except for the following.

The processing device 800 has a communication unit 802, a processor 804, an output unit 806, and a memory 808. The communication unit 802 is configured to cooperate with the connection unit 208 of the skin treatment device 100. The communication unit 802 is configured to receive the use data from the skin treatment device 100. The processor 804 is configured to adapt the user guidance based on the use data. The processor 804 is configured to generate a guidance signal based on the user guidance. The output unit 806 is configured to output the guidance signal, for example, to a display or any other type of device that allows the user to receive the user guidance in an understandable way.

The memory 808 comprises a computer program product comprising instructions, which when executed by the processor 804, cause the computer-implemented method of the first embodiment to be carried out. The processing device 800 comprises the processor 804 adapted to execute the computer program product that causes the computer-implemented method of the first embodiment to be carried out. For example, the memory 808 includes a default version of the user guidance. The processor 804 is configured to adapt the default version of the user guidance based on the use data.
For example, the processing device 800 is part of the mobile device 120.

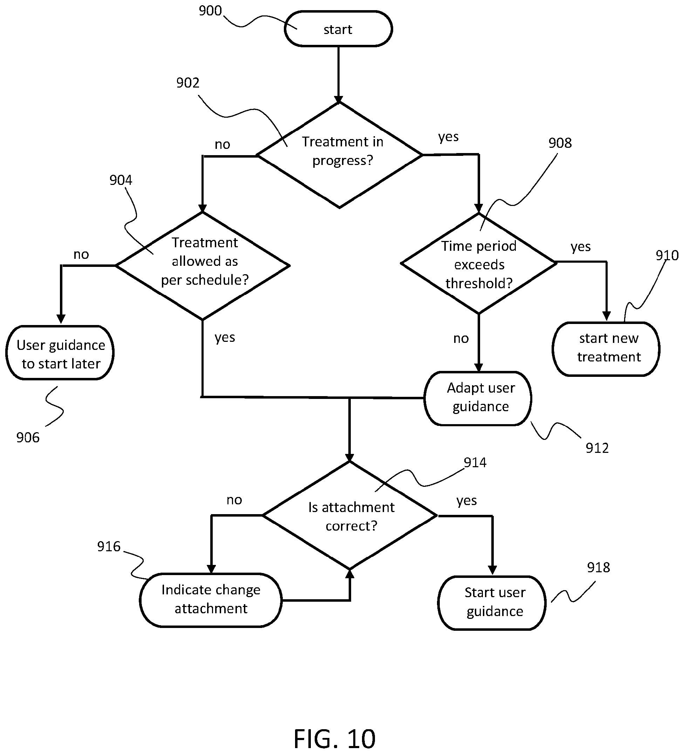

FIG. 10 depicts the computer-implemented method according to a third embodiment. The third embodiment has, for example, the same elements as the first embodiment or the second embodiment, except for the following.

In the third embodiment, the computer-implementation method starts at 900 when the connection 140 between the skin treatment device 100 and the processing device is re-established after the connection 140 has been interrupted.

At 902, the computer-implementation method determines whether a treatment is in progress. In case the previous treatment was completed, there is no treatment in progress, and the computer-implementation method continues to 904. At 904, the computer-implementation method determines whether a new treatment is allowed as per the schedule. The schedule is the treatment schedule that plans when treatments need to be performed to achieve a desired treatment result, and/or to prevent over-treatment of the skin. Over-treatment of the skin may lead to damage to the skin, discomfort or pain. In case the schedule does not allow a new treatment to start, the user guidance guides the user to start at a later time according to 906, for example, over one or more days later.

In case the treatment is allowed as per the schedule, the computer-implementation method continues to 914, where the computer-implementation method determines whether the correct attachment is attached to the skin treatment device 100. For example, the user inputs to the mobile device which attachment is attached to the skin treatment device, or the skin treatment device has an attachment sensor to detect which attachment is attached to the skin treatment device. For example, in case the treatment for the face is planned by the schedule, the computer-implementation method checks whether the attachment for the face is attached to the skin treatment device 100. In case another attachment is attached to the skin treatment device 100, such as an attachment for the arms or the legs, the computer-implementation method provides at 916 an indication to the user to change to the correct attachment. Then, the computer-implementation method re-assesses whether the correct attachment is attached to the skin treatment device 100 at 914. If the correct attachment is attached to the skin treatment device 100, the user guidance starts at 918.

In case the previous treatment was not completed, there is a treatment in progress, and the computer-implementation method goes from 902 to 908. At 908 the computer-implementation method determines whether the time period between the first point of time t1 and the second point of time t2 exceeds the threshold 500. In case the time period exceeds the threshold 500, a new treatment is started at 910. In case the time period does not exceed the threshold 500, the user guidance is adapted based on the treatment in progress.

Then, the computer-implementation method continues to 914, where the computer-implementation method determines whether the correct attachment is attached to the skin treatment device 100. In case another attachment is attached to the skin treatment device 100, such as an attachment for the arms or the legs, the computer-implementation method provides at 916 an indication to the user to change to the correct attachment. Then, the computer-implementation method re-assesses whether the correct attachment is attached to the skin treatment device 100 at 914. If the correct attachment is attached to the skin treatment device 100, the user guidance starts at 918.

For example, the user guidance started at 918 indicates to the user to move the skin treatment device 100 faster or slower across a body part. For example, the user guidance indicates to the user when to operate the user control to generate an IPL flash 201. For example, the user guidance indicates to the user in which direction to move the skin treatment device 100.

In a setting at home, the user handling the skin treatment device 100 places the head end 108 in contact with the user's own skin. In a commercial setting, such as a beauty salon or a spa, a professional user may handle the skin treatment device 100 and may place the head end 108 in contact with the skin of another person, e.g., a client of the beauty salon.

For example, the skin treatment is a non-therapeutic treatment, such as a non-therapeutic treatment of removing hairs with the skin treatment device 100. For example, the skin treatment is a cosmetic treatment for removing hairs with the skin treatment device 100. For example, the skin treatment is a non-therapeutic, cosmetic treatment for removing hairs with a light-based skin treatment device 100.

As discussed above, the device processor 202 or the processor 804 are adapted to perform the data processing. The processor or processor can be implemented in numerous ways, with software and/or hardware, to perform the various functions required. The device processor 202 or the processor 804 may employ one or more microprocessors that may be programmed using software (e.g. microcode) to perform the required functions. The device processor 202 or the processor 804 may be implemented as a combination of dedicated hardware to perform some functions and one or more programmed microprocessors and associated circuitry to perform other functions.

The processor or the processor may include circuitry. Examples of circuitry that may be employed include, but are not limited to, conventional microprocessors, application specific integrated circuits (ASICs), and field-programmable gate arrays (FPGAs). Thus, the processor or the processor may be embodied as a digital and/or analog processing system.

In various implementations, the device processor 202 or the processor 804 may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed or may be transportable, such that the one or more programs stored thereon can be loaded into the device processor 202 or the processor 804.

Functions implemented by the device processor 202 or the processor 804 may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A computer-implemented method of providing user guidance for a skin treatment device (100) to perform skin treatment, the computer-implemented method comprising:
establishing a connection (140) between the skin treatment device (100) and a processing device (800) performing the computer-implemented method;
determining a start of a treatment (T1) with the skin treatment device (100) at a first point of time (t1);
determining the connection (140) is interrupted after the first point of time (t1);
re-establishing the connection (140);
determining whether a time period between the first point of time (t1) and a second point of time (t2) exceeds a threshold (500),
wherein the second point of time (t2) is when the connection (140) has been re-established;
in case the time period does not exceed the threshold (500), receiving use data from the skin treatment device (100), and adapting the user guidance based on the use data;
in case the time period exceeds the threshold (500), not adapting the user guidance based on the use data,
wherein the use data is representative of one or more skin treatment sessions (S2) performed with the skin treatment device (100) while the connection was interrupted; and
generating a guidance signal representative of the user guidance.

2. The computer-implemented method of claim 1,
wherein each of the skin treatment sessions (S2) starts when the skin treatment device (100) is set to start to perform skin treatment based on at least one device setting, and
wherein each of the skin treatment sessions (S2) ends when at least one device setting changes.

3. The computer-implemented method of claim 2, wherein the at least one device setting comprises an on/off setting, an intensity level to perform the skin treatment, or an attachment attached to the skin treatment device (100).

4. The computer-implemented method of any one of the preceding claims,
wherein the skin treatment device (100) is an intense pulsed light (IPL) device adapted to perform IPL treatment by providing IPL flashes (201) to the skin,
wherein the use data comprises one or more of: a number of provided IPL flashes (201), a duration of the IPL treatment, or an intensity of the IPL flashes (201).

5. The computer-implemented method of any one of the preceding claims,
wherein the use data comprises an indication of an attachment used to perform the skin treatment,
wherein the attachment is one of multiple attachments adapted to connect to the skin treatment device (100),
wherein the multiple attachments are adapted to perform the skin treatment to different body parts.

6. The computer-implemented method of any one of the preceding claims,
wherein establishing the connection (140) comprises establishing the connection (140) via a wireless connection (140) between the skin treatment device (100) and the processing device (800).

7. The computer-implemented method of any one of the preceding claims,
wherein the processing device (800) is a mobile device (120).

8. The computer-implemented method of any one of the preceding claims,
wherein the threshold (500) is less than 48 hours, for example 24 hours.

9. The computer-implemented method of any one of the preceding claims,
wherein the user guidance comprises guidance (700) for the user about one or more of:
when to use the skin treatment device (100) to perform skin treatment;
on which body part to use the skin treatment device (100) to perform skin treatment;
which intensity setting to use; and
which attachment to use.

10. The computer-implemented method of any one of the preceding claims, comprising:
sending a reset signal to the skin treatment device (100),
wherein the reset signal causes the skin treatment device (100) to remove the use data from the skin treatment device (100).

11. The computer-implemented method of any one of the preceding claims, comprising:
receiving user input representative of whether the user wants to start a new treatment (T2);
in case the user input is representative of the user wanting to start the new treatment (T2), not adapting the user guidance based on the use data.

12. A skin treatment device (100) adapted to perform skin treatment, comprising:
a skin treatment unit (200) adapted to perform the skin treatment,
a use data collection unit (204) adapted to collect use data representative of one or more skin treatment sessions (S2) performed with the skin treatment device (100);
a use data storage unit (206) adapted to store the use data collected by the use collection unit;
a connection unit (208) adapted to connect to a processing device (800) adapted to perform the computer-implemented method according to any one of the preceding claims.

13. The skin treatment device according to claim 12, wherein the skin treatment device (100) is an intense pulsed light (IPL) device adapted to perform IPL treatment by providing IPL flashes (201) to the skin,
wherein the skin treatment unit (200) comprises an IPL light source adapted to generate the IPL flashes (201);
wherein the use data comprises one or more of: a number of provided IPL flashes (201), a duration of the IPL treatment, an intensity of the IPL flashes (201), and an indication of an attachment used to perform the IPL treatment,
wherein the attachment is one of multiple attachments adapted to connect to the IPL device,
wherein the multiple attachments are adapted to propagate the IPL flashes (201) to different body parts.

14. A computer program product comprising instructions, which when executed by a processor (804), cause the computer-implemented method of any one of claims 1-11 to be carried out.

15. A processing device (800), comprising a processor (804) adapted to execute the computer program product of claim 14.
